# EUROPÄISCHE PATENTSCHRIFT

(11) **EP 2 684 807 B1**
(45) Veröffentlichungstag und Bekanntmachung des Hinweises auf die Patenterteilung: **29.06.2016**
(21) Anmeldenummer: 12175691.0
(22) Anmeldetag: 10.07.2012
(51) Int. Cl.: B65D 1/02, A61C 19/00

(54) **Behälter für ein Reinigungs- oder Pflegemedium**
Container for a cleaning or maintaining agent
Récipient destiné à un milieu de nettoyage ou d'entretien

(43) Veröffentlichungstag der Anmeldung: 15.01.2014
(73) Patentinhaber: W & H Dentalwerk Bürmoos GmbH, 5111 Bürmoos (AT)
(72) Erfinder: Pfaffinger, Nikolaus, 5120 St. Pantaleon (AT); Schmied, Walter, 5121 Tarsdorf (AT); Galluseder, Florian, 5121 Tarsdorf (AT)
(74) Vertreter: Benda, Ralf

(56) Entgegenhaltungen:
- EP-A1- 2 138 127
- EP-A1- 2 322 116
- DE-U1- 9 310 601
- GB-A- 2 353 266
- US-A- 5 148 939
- US-A- 5 201 654
- US-A1- 2008 272 082

## Beschreibung

Die vorliegende Erfindung bezieht sich auf einen Behälter für ein Reinigungs- oder Pflegemedium und eine Reinigungs- oder Pflegevorrichtung mit einer Aufnahme für einen derartigen Behälter.

Ein Behälter für ein Reinigungs- oder Pflegemedium und eine Reinigungs- oder Pflegevorrichtung mit einer Aufnahme für einen derartigen Behälter sind zum Beispiel aus dem Patent US 5,057,283 bekannt. Die Behälter dienen zur Lagerung eines Reinigungs- oder Pflegemediums. Über eine Fördervorrichtung der Reinigungs- oder Pflegevorrichtung wird das Reinigungs- oder Pflegemedium aus den Behältern gefördert und dem zu reinigenden oder zu pflegenden Instrument zugeführt. Ist ein Behälter leer, so muss er entweder durch einen neuen, vollen Behälter ausgetauscht oder wieder befüllt werden. Oftmals sind die Fördervorrichtungen der Reinigungs- oder Pflegevorrichtungen und / oder die Behälter jedoch so ausgebildet, dass der Behälter nicht vollständig entleert wird. Dies ist verständlicherweise vom Anwender nicht erwünscht, da zum Beispiel, wenn der Behälter ausgetauscht und entsorgt wird, auch ein Teil des Reinigungs- oder Pflegemediums entsorgt wird, oder, wenn der Behälter wieder befüllt wird, das Befüllen öfter als notwendig durchgeführt werden muss.

Die Patentanmeldung GB 2 353 266 A offenbart einen Großbehälter für Trinkwasser, der nahe seinem Auslassstutzen eine plane Fläche aufweist, auf welcher der Großbehälter gekippt positioniert werden kann, um das Trinkwasser komfortabler ausschenken zu können.

Die Patentanmeldung US 2008/0272082 A1 zeigt verschiedene Ausführungsbeispiel von Arzneimittel- oder Getränkegebinden, die entweder eine schiefe Ebene im Gebindeinneren oder eine als schiefe Ebene ausgebildete Außenwand aufweisen, wodurch die Gebinde einfacher entleerbar sein sollen.

Der vorliegenden Erfindung liegt daher die Aufgabe zugrunde, einen Behälter für ein Reinigungs- oder Pflegemedium und eine Reinigungs- oder Pflegevorrichtung mit einer Aufnahme für einen derartigen Behälter zu schaffen, welche(r) die im Vorstehenden genannten Nachteile nicht aufweist. Insbesondere sollen der Behälter und die Reinigungs- oder Pflegevorrichtung derart beschaffen sein, dass eine möglichst vollständige Entleerung des Behälters möglich ist.

Diese Aufgabe wird durch einen Behälter für ein Reinigungs- oder Pflegemedium, insbesondere zur Reinigung oder Pflege eines medizinischen, dentalen oder chirurgischen Instruments, gemäß Anspruch 1 gelöst.

Durch das Anordnen des Auslassstutzens derart, dass die Ebene, entlang der sich die Bodenfläche erstreckt, den Auslassstutzen durchsetzt, ist zumindest ein Teil des Auslassstutzens tiefer angeordnet als die Bodenfläche des Behälters oder ist zumindest ein Teil des Auslassstutzens unterhalb der Bodenfläche des Behälters angeordnet oder bildet zumindest ein Teil des Auslassstutzens den tiefsten Punkt des Behälters (wenn der Behälter betriebsfertig in der Reinigungs- oder Pflegevorrichtung aufgenommen ist), so dass das, vorzugsweise flüssige, Reinigungs- oder Pflegemedium in den Auslassstutzen rinnt oder sich dort sammelt. Auf diese Weise ist es möglich, im Wesentlichen das gesamte Reinigungs- oder Pflegemedium aus dem Behälter zu fördern oder zumindest den im Behälter verbleibenden Rest des Reinigungs- oder Pflegemediums auf ein Minimum zu reduzieren.

Gemäß einem besonders bevorzugten Ausführungsbeispiel ist an der Bodenfläche des Behälters, insbesondere an der dem Behältervolumen zugewandten Innenseite der Bodenfläche, eine rinnenförmige Vertiefung vorgesehen ist, die mit dem Auslassstutzen verbunden ist. Diese rinnenförmige Vertiefung bewirkt eine zusätzliche Leitung des, vorzugsweise flüssigen, Reinigungs- oder Pflegemediums innerhalb des Behälters in Richtung des Auslassstutzens und / oder in den Auslassstutzen, wodurch der im Behälter verbleibende Rest des Reinigungs- oder Pflegemediums noch weiter reduziert wird.

Vorzugsweise umfasst der Auslassstutzen eine Mittelachse, wobei die Mittelachse und die Ebene, entlang der sich die Bodenfläche erstreckt, im Wesentlichen parallel zueinander angeordnet sind. Vorzugsweise weist der Auslassstutzen eine von der zumindest einen Seitenwand beabstandete Auslassöffnung für das Reinigungs- oder Pflegemedium auf, wobei die Ebene, entlang der sich die Bodenfläche erstreckt, die Auslassöffnung durchsetzt. Durch beide Maßnahmen wird insbesondere das Einbringen des Behälters in eine Reinigungs- oder Pflegevorrichtung, insbesondere in eine Aufnahme für den Behälter, und das gleichzeitige Anschließen des Behälters an die Fördervorrichtung zur Förderung des Reinigungs- oder Pflegemediums aus dem Behälter, insbesondere an eine Leitung der Fördervorrichtung, für den Anwender erleichtert: Das Einbringen des Behälters und das Anschließen des Behälters kann damit in einer einzigen, im Wesentlichen linearen, Schiebebewegung durch den Anwender erfolgen.

Bevorzugt umfasst der Auslassstutzen einen, insbesondere zylindrischen, Rohrstutzen. Der Auslassstutzen kann jedoch auch andere Formen aufweisen. Vorzugsweise ist am Auslassstutzen, insbesondere an der von der zumindest einen Seitenwand beabstandeten Auslassöffnung, ein Verschluss zum Verschließen des Behälters vorgesehen. Der Verschluss ist insbesondere beim oder durch das Anschließen des Behälters an die Fördervorrichtung zur Förderung des Reinigungs- oder Pflegemediums aus dem Behälter und / oder beim oder durch das Einbringen des Behälters in eine Reinigungs- oder Pflegevorrichtung öffenbar. Vorzugsweise ist der Verschluss durch die Fördervorrichtung oder ein mit der Fördervorrichtung verbundenes Bauteil, zum Beispiel durch ein Röhrchen oder eine Leitung oder eine Kanüle, öffenbar oder durchsetzbar. Bevorzugt umfasst der Verschluss eine Membran, besonders bevorzugt zusätzlich eine Metallmanschette, welche die Membran zumindest teilweise bedeckt.

Bevorzugt sind die Mittelachse des Auslassstutzens und die Ebene, entlang der sich die Bodenfläche erstreckt, versetzt zueinander oder beabstandet voneinander angeordnet. Vorzugsweise ist die Mittelachse weiter von dem Behältervolumen entfernt als die Ebene, entlang der sich die Bodenfläche erstreckt, oder es ist die Mittelachse unterhalb der Ebene, entlang der sich die Bodenfläche erstreckt, vorgesehen (wenn der Behälter betriebsfertig in der Reinigungs- oder Pflegevorrichtung aufgenommen ist). Diese tiefe Anordnung der Mittelachse des Auslassstutzens und damit des Auslassstutzens bewirkt in vorteilhafter Weise eine verbesserte Leitung des, vorzugsweise flüssigen, Reinigungs- oder Pflegemediums in den Auslassstutzen. Alternativ ist es auch möglich, dass die Mittelachse näher zu dem Behältervolumen angeordnet ist als die Ebene, entlang der sich die Bodenfläche erstreckt, oder dass die Mittelachse über der Ebene, entlang der sich die Bodenfläche erstreckt, vorgesehen ist (wenn der Behälter betriebsfertig in der Reinigungs- oder Pflegevorrichtung aufgenommen ist).

Vorzugsweise weist die Bodenfläche eine dem Behältervolumen zugewandte Innenseite und eine Außenseite auf, wobei die rinnenförmige Vertiefung an der Außenseite der Bodenfläche einen Vorsprung, insbesondere eine Wölbung, bildet. Dieser Vorsprung bildet insbesondere eine Führung an der Außenseite der Bodenfläche und erleichtert dadurch das Einbringen und / oder Positionieren des Behälters in eine Reinigungs- oder Pflegevorrichtung, insbesondere in eine Aufnahme für den Behälter, und insbesondere das Anschließen des Behälters an die Fördervorrichtung zur Förderung des Reinigungs- oder Pflegemediums aus dem Behälter, vorzugsweise an eine Leitung der Fördervorrichtung.

Vorzugsweise weist die rinnenförmige Vertiefung eine in Richtung des Auslassstutzens zunehmende Tiefe auf, wodurch ebenfalls eine verbesserte Leitung des, vorzugsweise flüssigen, Reinigungs- oder Pflegemediums in Richtung des Auslassstutzens oder in den Auslassstutzen erzielt wird. Zusätzlich oder alternativ weist der Vorsprung an der Außenseite der Bodenfläche eine in Richtung des Auslassstutzens (in Bezug auf die Bodenfläche) zunehmende Höhe auf.

Vorzugsweise weist der Behälter einen fließenden oder gleichmäßigen Übergang der rinnenförmigen Vertiefung in den Auslassstutzen auf, insbesondere einen fließenden oder gleichmäßigen Übergang der Außenwand der rinnenförmigen Vertiefung in die Außenwandung der Auslassstutzens. Damit kann das im Vorstehenden schon beschriebene Einbringen des Behälters in eine Reinigungs- oder Pflegevorrichtung und / oder das Anschließen des Behälters an die Fördervorrichtung zur Förderung des Reinigungs- oder Pflegemediums aus dem Behälter in einer gleichmäßigen, im Wesentlichen linearen, Schiebebewegung durch den Anwender erfolgen.

Vorzugsweise umfasst die rinnenförmige Vertiefung eine im Wesentlichen kreisbogenförmige, dem Behältervolumen zugewandte Innenwand und / oder eine im Wesentlichen kreisbogenförmige Außenwand, wodurch wiederum eine verbesserte Leitung des, vorzugsweise flüssigen, Reinigungs- oder Pflegemediums in Richtung des Auslassstutzens oder in den Auslassstutzen bzw. eine gleichmäßige, im Wesentlichen lineare, Schiebebewegung zum Einbringen des Behälters in eine Reinigungs- oder Pflegevorrichtung und / oder das Anschließen des Behälters an die Fördervorrichtung ermöglicht wird.

Bevorzugt weist der Behälter eine mit der zumindest einen Seitenwand verbundene Deckfläche auf, wobei zumindest Abschnitte der Deckfläche geneigt zur Bodenfläche und / oder zur Ebene, entlang der sich die Bodenfläche erstreckt, angeordnet sind. Die geneigte Deckfläche ermöglicht zum Beispiel eine bessere Handhabung des Behälters, insbesondere beim Einbringen oder Entnehmen aus der Reinigungs- oder Pflegevorrichtung oder eine bessere Ausnutzung des in der Reinigungs- oder Pflegevorrichtung vorhandenen Platzes, so dass ein Behälter mit einem größeren Volumen verwendbar ist. Die Deckfläche oder ein Abschnitt davon sind zum Beispiel in Richtung des Auslassstutzens und / oder in Richtung der zumindest einen Seitenwand geneigt.

Gemäß einem Ausführungsbeispiel ist eine Reinigungs- oder Pflegevorrichtung zur Reinigung oder Pflege eines, insbesondere medizinischen, dentalen oder chirurgischen, Instruments vorgesehen, die einen Behälter für ein Reinigungs- oder Pflegemedium und eine Fördervorrichtung zur Förderung des Reinigungs- oder Pflegemediums aus dem Behälter zu dem Instrument aufweist, wobei der Behälter eine Bodenfläche, die sich entlang einer Ebene erstreckt, zumindest eine mit der Bodenfläche verbundene Seitenwand, die mit der Bodenfläche ein Behältervolumen für das Reinigungs- oder Pflegemedium bildet, und einen Auslassstutzen, durch den das Reinigungs- oder Pflegemedium aus dem Behälter förderbar ist, umfasst, wobei der Auslassstutzen an der zumindest einen Seitenwand vorgesehen ist und die Ebene, entlang der sich die Bodenfläche erstreckt, den Auslassstutzen durchsetzt. Vorzugsweise ist der Behälter lösbar oder austauschbar mit der Reinigungs- oder Pflegevorrichtung verbunden. Vorzugsweise weist die Reinigungs- oder Pflegevorrichtung eine Aufnahme für den Behälter auf, in welcher der Behälter lösbar oder austauschbar einsetzbar ist und / oder in welcher der Behälter lagerbar ist.

Gemäß einem Ausführungsbeispiel ist eine Reinigungs- oder Pflegevorrichtung zur Reinigung oder Pflege eines, insbesondere medizinischen, dentalen oder chirurgischen, Instruments, vorgesehen, die umfasst: Ein Außengehäuse mit einer Basisfläche und zumindest einer damit verbundenen Seitenwand, eine Fördervorrichtung zur Förderung eines Reinigungs- oder Pflegemediums, insbesondere aus einem Behälter für ein Reinigungs- oder Pflegemedium, zu dem Instrument, eine Abgabevorrichtung zur Abgabe eines Reinigungs- oder Pflegemediums auf und / oder in das Instrument und zumindest eine Aufnahme für einen Behälter für ein Reinigungs- oder Pflegemedium, wobei die zumindest eine Aufnahme eine Grundfläche zur Lagerung der Bodenfläche des Behälters aufweist und wobei sich die Grundfläche entlang einer Ebene erstreckt, und wobei die Fördervorrichtung einen Leitungsabschnitt aufweist, der in den Auslassstutzen des Behälters einführbar ist, wobei der in den Auslassstutzen des Behälters einführbare Leitungsabschnitt in der Ebene, entlang der sich die Grundfläche erstreckt, oder zwischen der Ebene, entlang der sich die Grundfläche erstreckt, und der Basisfläche des Außengehäuses angeordnet ist.

Ein in die Aufnahme der Reinigungs- oder Pflegevorrichtung einbringbarer oder damit verbindbarer Behälter umfasst insbesondere: Eine Bodenfläche, wobei die Bodenfläche sich entlang einer Ebene erstreckt, zumindest eine mit der Bodenfläche verbundene Seitenwand, die mit der Bodenfläche ein Behältervolumen für das Reinigungs- oder Pflegemedium bildet, und einen Auslassstutzen, durch den das Reinigungs- oder Pflegemedium aus dem Behälter förderbar ist, wobei der Auslassstutzen an der zumindest einen Seitenwand vorgesehen ist und wobei die Ebene, entlang der sich die Bodenfläche erstreckt, den Auslassstutzen durchsetzt. Dieser Behälter und die Reinigungs- oder Pflegevorrichtung können vorzugsweise auch eine Reinigungs- oder Pflegeeinheit bilden.

Durch die Anordnung des in den Auslassstutzen des Behälters einführbaren Leitungsabschnitts in der Ebene, entlang der sich die Grundfläche erstreckt, oder zwischen der Ebene, entlang der sich die Grundfläche erstreckt, und der Basisfläche des Außengehäuses ist insbesondere eine Reinigungs- oder Pflegevorrichtung geschaffen, die mit dem Behälter verbindbar ist, dessen Auslassstutzen an der zumindest einen Seitenwand vorgesehen und von der Ebene, entlang der sich die Bodenfläche des Behälters erstreckt, durchsetzt ist. In anderen Worten ist der Leitungsabschnitt der Fördervorrichtung derart (tief) in Bezug auf die Aufnahme oder die Grundfläche der Aufnahme angeordnet, dass der Leitungsabschnitt in den (in Bezug auf die Bodenfläche des Behälters und / oder die Grundfläche der Aufnahme - wenn der Behälter betriebsfertig in der Reinigungs- oder Pflegevorrichtung aufgenommen ist - ebenfalls tief angeordneten) Auslassstutzen des Behälters einführbar ist. Insbesondere ist / sind somit durch das Vorsehen des in den Auslassstutzen des Behälters einführbaren Leitungsabschnitts in der Ebene, entlang der sich die Grundfläche erstreckt, oder zwischen der Ebene, entlang der sich die Grundfläche erstreckt, und der Basisfläche des Außengehäuses der Behälter und / oder der Auslassstutzen zumindest nahezu vollständig entleerbar.

Vorzugsweise ist in der Grundfläche der Aufnahme ein, insbesondere rinnenförmiger, Rücksprung zur Aufnahme der rinnenförmigen Vertiefung des Behälters vorgesehen, insbesondere zur Aufnahme des durch die rinnenförmige Vertiefung an der Außenseite der Bodenfläche gebildeten Vorsprungs oder Wölbung des Behälters. Wie im Vorstehenden bereits beschrieben wird damit das Einbringen des Behälters, insbesondere durch eine Schiebebewegung, in die Reinigungs- oder Pflegevorrichtung und / oder das Anschließen des Behälters an die Fördervorrichtung für den Anwender erleichtert.

Vorzugsweise weist der Rücksprung eine in Richtung des in den Auslassstutzen des Behälters einführbaren Leitungsabschnitts zunehmende Tiefe auf, welche insbesondere im Wesentlichen der zunehmenden Tiefe der rinnenförmigen Vertiefung des Behälters in Richtung des Auslassstutzens oder der zunehmenden Höhe des an der Außenseite der Bodenfläche gebildeten Vorsprungs oder Wölbung des Behälters entspricht.

Die Erfindung wird nachfolgend anhand bevorzugter Ausführungsbeispiele und Bezug nehmend auf die beigefügten Zeichnungen erläutert:
Figur 1 zeigt ein erstes Ausführungsbeispiel eines Behälters für ein Reinigungs- oder Pflegemedium.
Figur 2 zeigt eine Schnittdarstellung durch den Behälter der Figur 1.
Figur 3 ein zweites Ausführungsbeispiel eines Behälters für ein Reinigungs- oder Pflegemedium.
Figur 4 zeigt ein Ausführungsbeispiel einer Reinigungs- oder Pflegevorrichtung zur Reinigung oder Pflege eines, insbesondere medizinischen, dentalen oder chirurgischen, Instruments, mit einer Aufnahme für einen Behälter gemäß Figur 1 und einer Aufnahme für einen Behälter gemäß Figur 3.
Figur 5 zeigt einen vergrößerten Ausschnitt der Reinigungs- oder Pflegevorrichtung gemäß Figur 4 mit der Aufnahme für einen Behälter gemäß Figur 3.

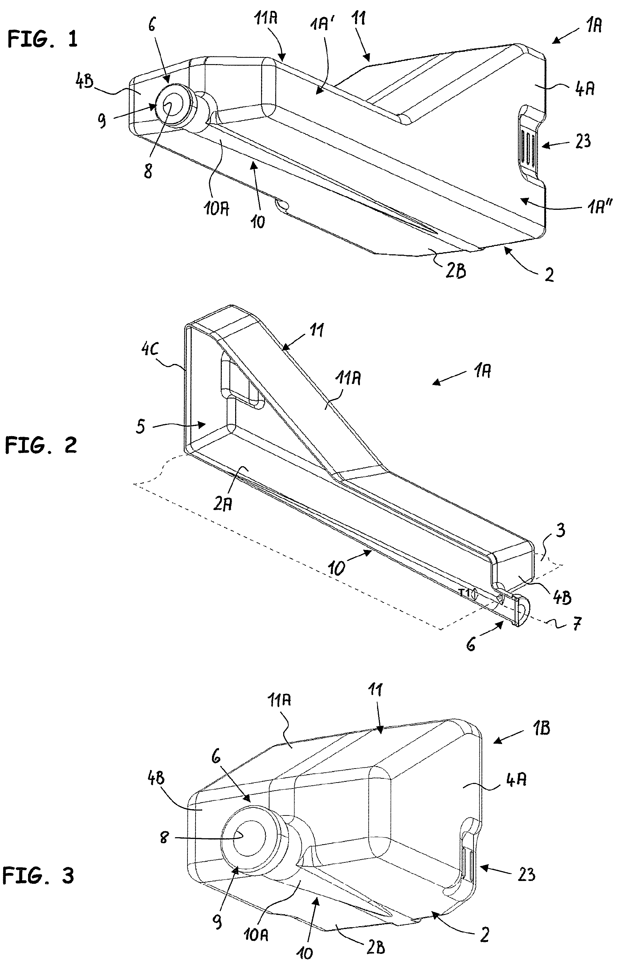

Die Figuren 1, 2 und 3 zeigen zwei unterschiedliche Ausführungsformen von Behältern 1A, 1 B für ein Reinigungs- oder Pflegemedium, insbesondere zur Reinigung oder Pflege eines medizinischen, dentalen oder chirurgischen Instruments. Gleiche Merkmale der beiden Behälter 1 A, 1 B sind im Folgenden gemeinsam beschrieben und weisen dieselben Bezugszeichen auf:
Die Behälter 1A, 1B weisen eine Bodenfläche 2 sowie zumindest eine, vorzugsweise mehrere, zum Beispiel vier Seitenwände 4A, 4B, 4C, auf. Die Seitenwände 4A, 4B, 4C sind fluiddicht miteinander und mit der Bodenfläche 2 verbunden, vorzugsweise integral oder einteilig miteinander und mit der Bodenfläche 2 verbunden. Die Bodenfläche 2 und die Seitenwände 4A, 4B, 4C bilden ein Behältervolumen 5 zur Aufnahme eines Reinigungs- oder Pflegemediums. Insbesondere aus der Figur 2 ist zu erkennen, dass sich die Bodenfläche 2 entlang einer Ebene 3 erstreckt. Die Bodenfläche 2 ist somit im Wesentlichen flach ausgebildet. Die Bodenfläche 2 weist des Weiteren eine dem Behältervolumen 5 zugewandte Innenseite 2A und eine Außenseite 2B auf.

Eine vorzugsweise fluiddicht mit der zumindest einen Seitenwand 4A, 4B, 4C verbundene, insbesondere integral oder einteilig mit der zumindest einen Seitenwand 4A, 4B, 4C hergestellte, Deckfläche 11 verschließt oder bedeckt die Behälter 1A, 1B oder das Behältervolumen 5. Zumindest ein Abschnitt 11A der Deckfläche 11 kann geneigt zur Bodenfläche 2 oder zur Ebene 3 angeordnet sein. Der Abschnitt 11A kann zum Beispiel in Richtung eines Auslassstutzens 6 des Behälters 1 A, 1 B und / oder von der Mitte oder einer Mittelachse der Deckfläche 11 in Richtung der zumindest einen Seitenwand 4A, 4B, 4C geneigt sein. Selbstverständlich kann die Deckfläche 11 oder zumindest ein Abschnitt der Deckfläche 11 auch im Wesentlichen parallel zur Bodenfläche 2 oder zur Ebene 3 ausgebildet sein.

Zumindest Teile der Behälter 1 A, 1 B, vorzugsweise die Bodenfläche 2, die zumindest eine Seitenwand 4A, 4B, 4C und die Deckfläche 11 sind aus Kunststoff hergestellt, zum Beispiel aus Polyethylen, insbesondere aus HD-PE.

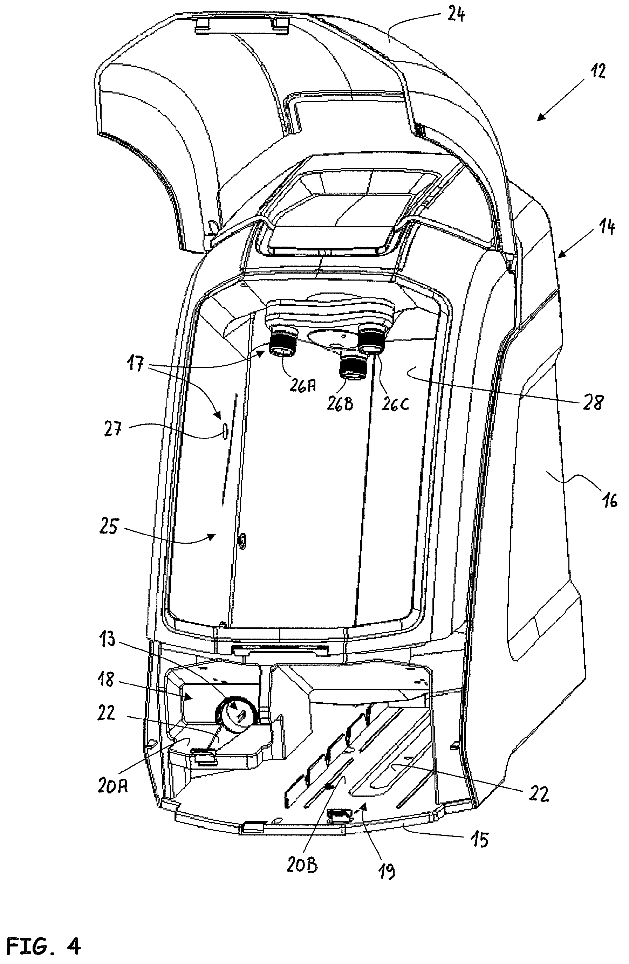

An der Bodenfläche 2 und / oder der Deckfläche 11 und / oder an der zumindest einen Seitenwand 4A, 4B, 4C ist eine Haltevorrichtung 23 vorgesehen, so dass die Behälter 1A, 1 B gut vom Anwender gehalten werden können, insbesondere beim Einstecken in oder Entnehmen aus eine(r) Reinigungs- oder Pflegevorrichtung 12 (siehe Figur 4). Die Haltevorrichtung 23 ist zum Beispiel als Rücksprung ausgebildet. Die Haltevorrichtung 23 weist zum Beispiel eine rauhe oder strukturierte Oberfläche auf.

Der Auslassstutzen 6, durch den das Reinigungs- oder Pflegemedium aus dem Behälter 1A, 1B förderbar ist, ist an der zumindest einen Seitenwand 4A, 4B, 4C des Behälters 1A, 1B vorgesehen. Der Auslassstutzen 6 ist hohl oder weist eine mit dem Behältervolumen 5 verbundene Bohrung auf und ist zum Beispiel als zylindrischer Stutzen oder Rohrstutzen ausgebildet. Der Auslassstutzen 6 umfasst somit ein erstes Ende, das mit der zumindest einen Seitenwand 4A, 4B, 4C fluiddicht, insbesondere unlösbar und / oder integral, verbunden ist, und ein zweites, von der zumindest einen Seitenwand 4A, 4B, 4C beabstandetes zweites, freies Ende auf. Wie insbesondere aus der Figur 2 zu erkennen ist, durchsetzt die Ebene 3, entlang der sich die Bodenfläche 2 erstreckt, den Auslassstutzen 6. Der Auslassstutzen 6 ist somit beidseitig zur Ebene 3 angeordnet. Zumindest Teile des Auslassstutzens 6 sind daher von dem Behältervolumen 5 weiter entfernt als die Ebene 3 oder in Bezug auf das Behältervolumen 5 unterhalb der Ebene 3 angeordnet.

Der Auslassstutzen 6 weist eine Mittelachse 7 auf, wobei die Mittelachse 7 und die Ebene 3, entlang der sich die Bodenfläche 2 erstreckt, vorzugsweise im Wesentlichen parallel zueinander angeordnet sind. Insbesondere sind die Mittelachse 7 und die Ebene 3 versetzt zueinander angeordnet. Vorzugsweise ist die Mittelachse 7 in etwa in der Ebene 3 angeordnet oder die Ebene 3 ist zwischen dem Behältervolumen 5 und der Mittelachse 7 angeordnet, so dass die Mittelachse 7 in Bezug auf das Behältervolumen 5 unterhalb der Ebene 3 liegt.

Der Auslassstutzen umfasst an seinem freien Ende eine von der zumindest einen Seitenwand 4A, 4B, 4C beabstandete Auslassöffnung 8 zur Abgabe des Reinigungs- oder Pflegemediums. Vorzugsweise durchsetzt die Ebene 3, entlang der sich die Bodenfläche 2 erstreckt, die Auslassöffnung 8, so dass die Auslassöffnung 8 insbesondere beidseitig zur Ebene 3 angeordnet ist. Am Auslassstutzen 6, insbesondere an der Auslassöffnung 8, ist des Weiteren ein Verschluss 9, vorzugsweise eine Membran, zum Verschließen des Behälters 1A, 1B vorgesehen. Der Verschluss 9 umfasst auch eine Metallmanschette, welche die Membran zumindest teilweise bedeckt.

In der Bodenfläche 2, insbesondere an der Innenseite 2A der Bodenfläche 2, ist des Weiteren eine rinnenförmige Vertiefung 10 vorgesehen, die mit dem Auslassstutzen 6 verbunden ist. Die rinnenförmige Vertiefung 10 erstreckt sich zumindest über einen Teil der Bodenfläche 2, vorzugsweise über einen Großteil der Bodenfläche 2. Insbesondere erstreckt sich die rinnenförmige Vertiefung 10 von dem Auslassstutzen 6 in Richtung oder in die Nähe jener Seitenwand 4C, die gegenüber der Seitenwand 4B angeordnet ist, an welcher der Auslassstutzen 6 vorgesehen oder befestigt ist. Die rinnenförmige Vertiefung 10 ist im Wesentlichen gerade ausgebildet, sie kann jedoch auch gebogen sein. Die rinnenförmige Vertiefung 10 umfasst zumindest einen Kanal oder Hauptkanal, der mit dem Auslassstutzen 6 verbunden ist oder in diesen mündet. Wahlweise umfasst die rinnenförmige Vertiefung 10 mehrere miteinander verbundene Kanäle, insbesondere einen Hauptkanal, der mit dem Auslassstutzen 6 verbunden ist und der mit einem oder mehreren Seitenkanälen verbunden ist, wobei der Seitenkanal insbesondere in den Hauptkanal mündet. Vorzugsweise bildet die rinnenförmige Vertiefung 10 des Weiteren an der Außenseite 2B der Bodenfläche 2 einen Vorsprung 10A, insbesondere eine Wölbung.

Des Weiteren weist die der rinnenförmigen Vertiefung 10 eine in Richtung des Auslassstutzens 6 zunehmende Tiefe T1 auf (siehe Figur 2). Die Tiefe T1 beträgt im Bereich nahe des Auslassstutzens 6 zum Beispiel in etwa 3 - 5 mm, vorzugsweise etwa 4 mm. Vorzugsweise nimmt auch die Breite oder lichte Weite der rinnenförmigen Vertiefung 10 in Richtung des Auslassstutzens 6 zu. Insbesondere entspricht die lichte Weite der rinnenförmigen Vertiefung 10 im Bereich nahe des Auslassstutzens 6 in etwa oder genau dem Durchmesser der Bohrung des Auslassstutzens 6. Die lichte Weite der rinnenförmigen Vertiefung 10 im Bereich nahe des Auslassstutzens 6 beträgt etwa 8 - 14 mm, vorzugsweise etwa 11 mm.

Der Übergang der rinnenförmigen Vertiefung 10 in den Auslassstutzen 6, insbesondere der Übergang der Außenwand der rinnenförmigen Vertiefung 10 in die Außenwandung des Auslassstutzens 6, ist fließend oder gleichmäßig ausgebildet. Die (dem Behältervolumen 5 zugewandte) Innenwand und / oder die Außenwand der rinnenförmigen Vertiefung 10 ist/sind im Wesentlichen kreisbogenförmig ausgebildet.

Gemäß einem Ausführungsbeispiel sind die Behälter 1A, 1B wieder befüllbar und weisen, zum Beispiel an der Deckfläche 11, eine verschließbare Öffnung zum Befüllen auf.

Die Behälter 1A und 1B unterscheiden sich vor allem in ihrer Form und in der Größe ihrer Behältervolumina. Der Behälter 1A umfasst ein Volumen von 1000 ml, und weist einen schmalen, annähernd quaderförmigen Vorderabschnitt 1A' mit dem Auslassstutzen 6 und einen damit verbundenen rückwärtigen, im Querschnitt dreieckigen Abschnitt 1A" auf. Zumindest ein Teil der Deckfläche 11 des Abschnitts 1A" ist in Richtung des Auslassstutzens 6 geneigt. Zumindest ein Teil der Deckfläche 11 des Abschnitts 1A' ist in Richtung der Seitenwand 4A geneigt. Der Behälter 1A ist insbesondere zur Aufnahme eines Reinigungs- oder Desinfektionsmittels vorgesehen.

Der Behälter 1 B umfasst ein Volumen von etwa 200 ml. Zumindest ein Teil der Deckfläche 11 des Behälters 1 B ist in Richtung des Auslassstutzens 6 geneigt. Der Behälter 1 B ist insbesondere zur Aufnahme eines Schmiermittels oder Öls vorgesehen.

Die Figur 4 zeigt eine Reinigungs- oder Pflegevorrichtung 12 zur Reinigung oder Pflege eines, insbesondere medizinischen, dentalen oder chirurgischen, Instruments. Die Reinigungs- oder Pflegevorrichtung 12 umfasst ein, bevorzugt aus Kunststoff hergestelltes, Außengehäuse 14 mit einer Basisfläche oder Bodenplatte 15 und mehreren Seitenwänden 16. Die Reinigungs- oder Pflegevorrichtung 12 weist des Weiteren eine bewegliche, insbesondere verschwenkbare, Türe oder einen beweglichen, insbesondere verschwenkbaren, Deckel 24 auf, der bevorzugt über Scharniere am Außengehäuse 14 bewegbar ist. Mit dem Deckel 24 ist eine im Inneren des Außengehäuses 14 angeordnete Reinigungs- oder Pflegekammer 25 verschließbar, in der zumindest eine, vorzugsweise mehrere Kupplungen oder Anschlüsse, im vorliegenden Fall drei Anschlüsse 26A, 26B, 26C, für zu reinigende oder zu pflegende medizinische, insbesondere dentale, Instrumente vorgesehen sind. Mittels der Anschlüsse 26A, 26B, 26C sind ein oder mehrere Reinigungs- oder Pflegemittel in das Innere der Instrumente förderbar. Zusätzlich befinden sich in der Reinigungs- oder Pflegekammer 25 eine oder mehrere Öffnungen oder Düsen 27, über die ein Reinigungs- oder Pflegemittel auf die Außenseite der Instrumente abgegeben werden kann. Die Düsen 27 sind von den Anschlüssen 26A, 26B, 26C getrennt angeordnet und, insbesondere zeitlich, unabhängig von den Anschlüssen 26A, 26B, 26C mit einem Reinigungs- und/oder Pflegemittel versorgbar. Die zumindest eine Düse 27 und die Anschlüsse 26A, 26B, 26C sind Teil einer Abgabevorrichtung 17 zur Abgabe eines Reinigungs- oder Pflegemediums auf und / oder in zumindest ein Instrument oder mit einer derartigen Abgabevorrichtung 17 verbunden.

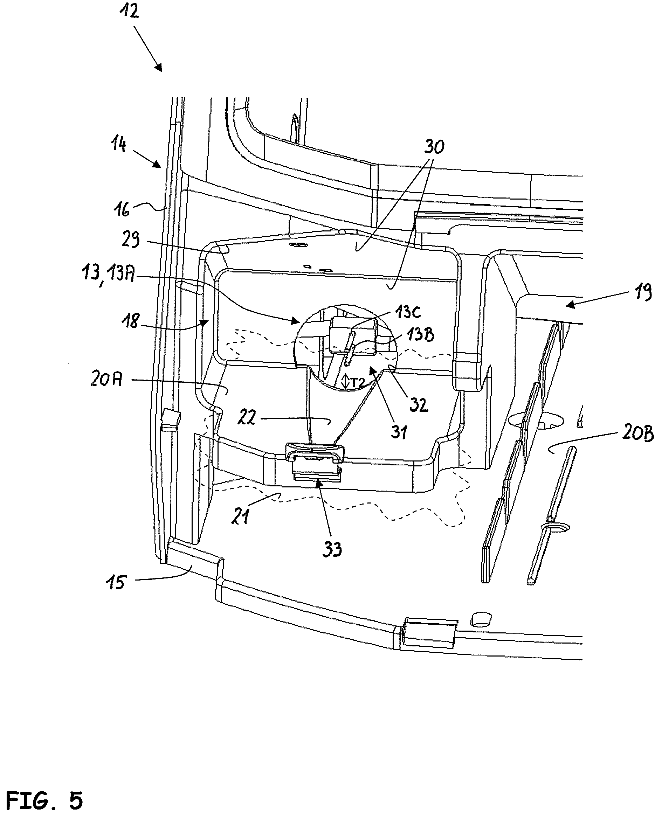

Die Reinigungs- oder Pflegekammer 25 wird durch eine im Inneren des Reinigungs- oder Pflegegeräts 12 angeordnete Trennwand 28 von einem Steuerraum 31 (siehe Figur 5) getrennt. Am oder im Steuerraum 31 sind insbesondere eine Vielzahl von Steuer- und Funktionselementen des Reinigungs- oder Pflegegeräts 12 vorgesehen, zum Beispiel eine Versorgungs- oder Fördervorrichtung 13 zur Förderung eines Reinigungs- oder Pflegemediums zu dem Instrument, von der in der Figur 5 beispielhaft ein kurzes Leitungsstück 13A erkennbar ist und die unter anderem einen Anschluss 13B, 13C an zumindest einen Behälter 1 A, 1 B und vorzugsweise an eine oder mehrere Fluidquellen, zum Beispiel an eine Druckluft- und oder Wasserquelle, Leitungen 13A zur Förderung der Fluide, Reinigungs-, Pflege- oder Schmiermittel, Pumpen, Steuer- und Stellmittel, zum Beispiel Ventile, Drosseln, Sensoren, zum Beispiel zur Durchflussmessung, Konzentrationsmessung, Mischer und viele weitere Bauteile enthalten kann. Neben der Fördervorrichtung 13 können am oder im Steuerraum 31 weitere Bauteile, wie zum Beispiel ein Anschluss an eine Energiequelle, Auffangwannen oder Ablässe für gebrauchte Reinigungs- oder Pflegemittel, eine Steuervorrichtung zur Steuerung und / oder Regelung der Reinigungs- oder Pflegeverfahren, eine Speichereinheit, zum Beispiel zur Speicherung von Betriebsdaten, eine Antriebseinheit zum Bewegen der Anschlüsse 26A, 26B, 26C, eine Betriebsanzeige, ein Bedienpanel für den Anwender oder zumindest Teile einer Detektionseinheit zur Bestimmung welcher der Anschlüsse 26A, 26B, 26C mit einem medizinischen, insbesondere dentalen, Instrument belegt ist und / oder welcher der Anschlüsse 26A, 26B, 26C nicht mit einem Instrument belegt ist, vorgesehen sein.

Die Reinigungs- oder Pflegevorrichtung 12 weist des Weiteren zumindest eine Kammer oder Aufnahme 18, 19 zur lösbaren Aufnahme eines Behälters 1A, 1B für ein Reinigungs- oder Pflegemedium auf (siehe auch Figur 5). Die zumindest eine Aufnahme 18, 19 ist zum Beispiel unterhalb der Reinigungs- oder Pflegekammer 25 vorgesehen. Die Kammer 18 ist insbesondere für die Aufnahme des Behälters 1 B und die Kammer 19 insbesondere für die Aufnahme des Behälters 1A vorgesehen. Dem entsprechend sind die beiden Kammern 18, 19 unterschiedlich geformt und weisen unterschiedliche Volumina auf.

Die Aufnahmen 18, 19 umfassen jeweils eine Grundfläche 20A, 20B zur Lagerung der Bodenfläche 2 des Behälters 1A, 1 B, wobei sich die Grundflächen 20A, 20B jeweils entlang einer Ebene 21 erstrecken. Die Aufnahmen 18, 19 weisen des Weiteren eine Öffnung 29 zum Einbringen und Entfernen der Behälter 1 A, 1B und eine oder mehrere Wände 30 auf, welche die Aufnahmen 18, 19 begrenzen. An einer dieser Wände 30, vorzugsweise an jener Wand 30, die gegenüber der Öffnung 29 angeordnet ist, ist ein Durchbruch 32 vorgesehen, so dass eine Verbindung zwischen der Kammer 18 und dem Steuerraum 31, vorzugsweise der Fördervorrichtung 13, insbesondere über den Leitungsabschnitt 13A mit den Röhrchen 13B, 13C, gebildet ist.

Die Fördervorrichtung 13 oder der Leitungsabschnitt 13A umfassen zumindest ein, vorzugsweise metallisches, Röhrchen 13B, das in den Auslassstutzen 6 des Behälters 1A, 1B einführbar ist. Durch dieses Röhrchen 13B ist das in dem Behälter 1 A, 1B enthaltene Reinigungs- oder Pflegemedium mittels und durch die Fördervorrichtung 13 dem zu reinigenden oder pflegenden Instrument zuführbar. Bevorzugt umfassen die Fördervorrichtung 13 oder der Leitungsabschnitt 13A zumindest ein weiteres, vorzugsweise metallisches, Röhrchen 13C, das in den Auslassstutzen 6 des Behälters 1 A, 1B einführbar ist, durch welches ein Fördermittel, zum Beispiel Druckluft, in den Behälter 1 A, 1 B förderbar ist, wodurch das Reinigungs- oder Pflegemedium aus dem Behälter 1 A, 1B getrieben wird. Demgemäß ist das Röhrchen 13C mit einer Quelle für das Fördermittel verbindbar. Der Leitungsabschnitt 13A, insbesondere die beiden Röhrchen 13B, 13C, sind derart ausgebildet, dass sie den Verschluss 9, insbesondere dessen Membran durchdringen können. Die beiden Röhrchen 13B, 13C sind insbesondere derart angeordnet, dass ein Röhrchen, vorzugsweise das Reinigungs- oder Pflegemedium leitende Röhrchen 13B, näher zur Basisfläche 15 angeordnet ist als das andere Röhrchen, vorzugsweise das Fördermittel leitende Röhrchen 13C. Besonders bevorzugt sind die beiden Röhrchen 13B, 13C auf einer zur Basisfläche 15 im Wesentlichen senkrecht angeordneten Achse übereinander angeordnet.

Aus der Figur 5 ist zu erkennen, dass der in den Auslassstutzen 6 des Behälters 1 A, 1 B einführbare Leitungsabschnitt 13A, vorzugsweise zumindest eines der beiden Röhrchen 13B, 13C, insbesondere jenes Röhrchen 13B, durch welches das in dem Behälter 1A, 1 B enthaltene Reinigungs- oder Pflegemedium dem Instrument zuführbar ist, in der Ebene 21, entlang der sich die Grundfläche 20A, 20B erstreckt, oder zwischen der Ebene 21 und der Basisfläche 15 des Außengehäuses 14 angeordnet ist.

In der Grundfläche 20A, 20B der Aufnahme 18, 19 ist des Weiteren ein, insbesondere rinnenförmiger, Kanal oder Rücksprung 22 zur Aufnahme der rinnenförmigen Vertiefung 10 des Behälters 1 A, 1 B vorgesehen, insbesondere zur Aufnahme des durch die rinnenförmige Vertiefung 10 an der Außenseite 2B der Bodenfläche 2 gebildeten Vorsprungs oder Wölbung 10A. Vorzugsweise hat der Rücksprung 22 eine in Richtung des Leitungsabschnitts 13A - 13C zunehmende Tiefe T2, insbesondere auch eine in Richtung des Leitungsabschnitts 13A - 13C zunehmende Breite. Bevorzugt weisen der Rücksprung 22 und die rinnenförmigen Vertiefung 10 des Behälters 1A, 1B im Wesentlichen einander ergänzende Formen und / oder Abmessungen auf.

An der Aufnahme 18, 19, zum Beispiel an der Grundfläche 20A, 20B, ist eine Fixiervorrichtung 33 vorgesehen, um den Behälter 1A, 1 B in der Aufnahme 18, 19 lösbar zu befestigen. Die Fixiervorrichtung 33 ist zum Beispiel als federnde Klammer ausgebildet. Die Aufnahmen 18, 19 sind durch einen abnehmbaren oder verschwenkbar an der Reinigungs- oder Pflegevorrichtung 12 befestigten Deckel (nicht dargestellt) verschließbar.

Die Erfindung ist nicht auf das beschriebene Ausführungsbeispiel beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Behälter (1A, 1B) für ein Reinigungs- oder Pflegemedium, insbesondere zur Reinigung oder Pflege eines medizinischen, dentalen oder chirurgischen Instruments, wobei der Behälter (1A,1B) in die Aufnahme einer Reinigungs- oder Pflegevorrichtung einbringbar ist und wobei der Behälter (1A, 1B) umfasst: Eine Bodenfläche (2), wobei die Bodenfläche (2) sich entlang einer Ebene (3) erstreckt, zumindest eine mit der Bodenfläche (2) verbundene Seitenwand (4A, 4B, 4C), die mit der Bodenfläche (2) ein Behältervolumen (5) für das Reinigungs- oder Pflegemedium bildet, und einen Auslassstutzen (6), durch den das Reinigungs- oder Pflegemedium aus dem Behälter (1A, 1B) förderbar ist, wobei der Auslassstutzen (6) an der zumindest einen Seitenwand (4A, 4B, 4C) vorgesehen ist und wobei die Ebene (3), entlang der sich die Bodenfläche (2) erstreckt, den Auslassstutzen (6) durchsetzt, **dadurch gekennzeichnet, dass**
der Auslassstutzen (6) eine von der zumindest einen Seitenwand (4A, 4B, 4C) beabstandete Auslassöffnung (8) für das Reinigungs- oder Pflegemedium aufweist, wobei die Ebene (3), entlang der sich die Bodenfläche (2) erstreckt, die Auslassöffnung (8) durchsetzt.

2. Behälter (1A, 1B) für ein Reinigungs- oder Pflegemedium nach Anspruch 1, **dadurch gekennzeichnet, dass**
der Auslassstutzen (6) eine Mittelachse (7) umfasst, wobei die Mittelachse (7) und die Ebene (3), entlang der sich die Bodenfläche (2) erstreckt, im Wesentlichen parallel zueinander angeordnet sind.

3. Behälter (1A, 1 B) für ein Reinigungs- oder Pflegemedium nach Anspruch 1 oder 2, **dadurch gekennzeichnet, dass**
der Auslassstutzen (6) eine Mittelachse (7) umfasst, wobei die Mittelachse (7) und die Ebene (3), entlang der sich die Bodenfläche (2) erstreckt, versetzt zueinander angeordnet sind.

4. Behälter (1A, 1B) für ein Reinigungs- oder Pflegemedium nach einem der vorstehenden Ansprüche, **dadurch gekennzeichnet, dass**
die Mittelachse (7) des Auslassstutzens (6) in etwa in der Ebene (3) angeordnet ist oder die Ebene (3) ist zwischen dem Behältervolumen (5) und der Mittelachse (7) angeordnet, so dass die Mittelachse (7) in Bezug auf das Behältervolumen (5) unterhalb der Ebene (3) liegt.

5. Behälter (1A, 1B) für ein Reinigungs- oder Pflegemedium nach einem der vorstehenden Ansprüche, **dadurch gekennzeichnet, dass**
am Auslassstutzen (6), insbesondere an der von der zumindest einen Seitenwand (4A, 4B, 4C) beabstandeten Auslassöffnung (8), ein Verschluss (9), vorzugsweise eine Membran, zum Verschließen des Behälters (1A, 1 B) vorgesehen ist.

6. Behälter (1A, 1B) für ein Reinigungs- oder Pflegemedium nach einem der vorstehenden Ansprüche, **dadurch gekennzeichnet, dass** an der Bodenfläche (2) eine rinnenförmige Vertiefung (10) vorgesehen ist, die mit dem Auslassstutzen (6) verbunden ist.

7. Behälter (1A, 1 B) für ein Reinigungs- oder Pflegemedium nach Anspruch 6, **dadurch gekennzeichnet, dass**
die Bodenfläche (2) eine dem Behältervolumen (5) zugewandte Innenseite (2A) und eine Außenseite (2B) aufweist, wobei die rinnenförmige Vertiefung (10) an der Außenseite (2B) der Bodenfläche (2) einen Vorsprung (10A), insbesondere eine Wölbung, bildet.

8. Behälter (1A, 1 B) für ein Reinigungs- oder Pflegemedium nach Anspruch 6 oder 7, **gekennzeichnet durch**
eine in Richtung des Auslassstutzens (6) zunehmende Tiefe (T1) der rinnenförmigen Vertiefung (10).

9. Behälter (1A, 1 B) für ein Reinigungs- oder Pflegemedium nach einem der Ansprüche 6 - 8, **gekennzeichnet durch**
einen fließenden oder gleichmäßigen Übergang der rinnenförmigen Vertiefung (10) in den Auslassstutzen (6), insbesondere **durch** einen fließenden oder gleichmäßigen Übergang der Außenwand der rinnenförmigen Vertiefung (10) in die Außenwandung der Auslassstutzens (6).

10. Behälter (1A, 1B) für ein Reinigungs- oder Pflegemedium nach einem der Ansprüche 6 - 8, **gekennzeichnet durch**
eine im Wesentlichen kreisbogenförmige, dem Behältervolumen (5) zugewandte Innenwand der rinnenförmigen Vertiefung (10) und / oder eine im Wesentlichen kreisbogenförmige Außenwand der rinnenförmigen Vertiefung (10).

11. Behälter (1A, 1B) für ein Reinigungs- oder Pflegemedium nach einem der vorstehenden Ansprüche, **gekennzeichnet durch**
eine mit der zumindest einen Seitenwand (4A, 4B, 4C) verbundene Deckfläche (11), wobei zumindest ein Abschnitt (11A) der Deckfläche (11) geneigt zur Bodenfläche (2) und / oder zur Ebene (3), entlang der sich die Bodenfläche (2) erstreckt, angeordnet sind.

12. Reinigungs- oder Pflegevorrichtung (12) zur Reinigung oder Pflege eines, insbesondere medizinischen, dentalen oder chirurgischen, Instruments, mit einem Behälter (1A, 1B) für ein Reinigungs- oder Pflegemedium nach einem der vorstehenden Ansprüche und einer Fördervorrichtung (13) zur Förderung des Reinigungs- oder Pflegemediums aus dem Behälter (1A, 1 B) zu dem Instrument.

13. Reinigungs- oder Pflegevorrichtung (12) zur Reinigung oder Pflege eines, insbesondere medizinischen, dentalen oder chirurgischen, Instruments, nach Anspruch 12 **gekennzeichnet durch** ein Außengehäuse (14) mit einer Basisfläche (15) und zumindest einer damit verbundenen Seitenwand (16), **durch** eine Abgabevorrichtung (17) zur Abgabe eines Reinigungs- oder Pflegemediums auf und / oder in das Instrument und **durch** zumindest eine Aufnahme (18, 19) für den Behälter (1A, 1B) für ein Reinigungs- oder Pflegemedium wobei die zumindest eine Aufnahme (18, 19) eine Grundfläche (20A, 20B) zur Lagerung der Bodenfläche (2) des Behälters (1A, 1B) aufweist und wobei sich die Grundfläche (20A, 20B) entlang einer Ebene (21 ) erstreckt, wobei die Fördervorrichtung (13) einen Leitungsabschnitt (13A - 13C) aufweist, der in den Auslassstutzen (6) des Behälters (1A, 1B) einführbar ist, wobei der in den Auslassstutzen (6) des Behälters (1A, 1B) einführbare Leitungsabschnitt (13A - 13C) in der Ebene (21), entlang der sich die Grundfläche (20A, 20B) erstreckt, oder zwischen der Ebene (21), entlang der sich die Grundfläche (20A, 20B) erstreckt, und der Basisfläche (15) des Außengehäuses (14) angeordnet ist.

14. Reinigungs- oder Pflegevorrichtung (12) nach Anspruch 13, **dadurch gekennzeichnet, dass** in der Grundfläche (20A, 20B) der Aufnahme (18, 19) ein, insbesondere rinnenförmiger, Rücksprung (22) zur Aufnahme der rinnenförmigen Vertiefung (10) des Behälters (1A, 1B) vorgesehen ist, insbesondere zur Aufnahme des durch die rinnenförmige Vertiefung (10) an der Außenseite (2B) der Bodenfläche (2) gebildeten Vorsprungs oder Wölbung (10A) des Behälters (1A, 1 B).

15. Reinigungs- oder Pflegevorrichtung (12) nach Anspruch 14, **gekennzeichnet durch** eine in Richtung des in den Auslassstutzen (6) des Behälters (1A, 1B) einführbaren Leitungsabschnitts (13A - 13C) zunehmende Tiefe (T2) des Rücksprungs (22).

## Claims

1. A container (1A, 1 B) for a cleaning or care medium, in particular for cleaning or care of a medical, dental or surgical instrument, wherein the container (1A,1B) can be introduced into the receptacle of a cleaning or care device and wherein the container (1A, 1B) comprises: a bottom surface (2), wherein the bottom surface (2) extends along a plane (3), at least one side wall (4A, 4B, 4C) which is connected to the bottom surface (2) and together with the bottom surface (2) forms a container volume (5) for the cleaning or care medium, and an outlet connector (6) through which the cleaning or care medium can be conveyed out of the container (1A, 1 B), wherein the outlet connector (6) is provided on the at least one side wall (4A, 4B, 4C) and wherein the plane (3) along which the bottom surface (2) extends passes through the outlet connector (6), **characterized in that**
the outlet connector (6) comprises an outlet opening (8) for the cleaning or care medium at a distance from the at least one side wall (4A, 4B, 4C), wherein the plane (3) along which the bottom surface (2) extends passes through the outlet opening (8).

2. The container (1A, 1B) for a cleaning or care medium according to claim 1, **characterized in that**
the outlet connector (6) comprises a central axis (7), wherein the central axis (7) and the plane (3) along which the bottom surface (2) extends are arranged essentially parallel to one another.

3. The container (1A, 1B) for a cleaning or care medium according to claim 1 or 2, **characterized in that**
the outlet connector (6) comprises a central axis (7), wherein the central axis (7) and the plane (3) along which the bottom surface (2) extends are arranged with an offset from one another.

4. The container (1A, 1B) for a cleaning or care medium according to any one of the preceding claims, **characterized in that**
the central axis (7) of the outlet connector (6) is arranged approximately in the plane (3) or the plane (3) is arranged between the container volume (5) and the central axis (7) so that the central axis (7) is situated beneath the plane (3) with respect to the container volume (5).

5. The container (1A, 1B) for a cleaning or care medium according to any one of the preceding claims, **characterized in that**
a closure (9), preferably a diaphragm, for sealing the container (1A, 1B) is provided on the outlet connector (6), in particular on the outlet opening (8) which is at a distance from the at least one side wall (4A, 4B, 4C).

6. The container (1A, 1B) for a cleaning or care agent according to any one of the preceding claims, **characterized in that**
a channel-shaped recess (10) which is connected to the outlet connector (6) is provided on the bottom surface (2).

7. The container (1A, 1B) for a cleaning or care medium according to claim 6, **characterized in that**
the bottom surface (2) comprises an inner surface (2A) facing the container volume (5) and an outer surface (2B), wherein the channel-shaped recess (10) forms a protrusion (10A), in particular a bulge on the outer surface (2B) of the bottom surface (2).

8. The container (1A, 1B) for a cleaning or care medium according to claim 6 or 7, **characterized by**
an increasing depth (T1) of the channel-shaped recess (10) in the direction of the outlet connector (6).

9. The container (1A, 1 B) for a cleaning or care medium according to any one of claims 6 - 8, **characterized by**
a smooth or even transition of the channel-shaped recess (10) into the outlet connector (6), in particular through a smooth or even transition of the outer wall of the channel-shaped recess (10) into the outside wall of the outlet connector (6).

10. The container (1A, 1B) for a cleaning or care medium according to any one of claims 6 - 8, **characterized by**
an essentially arc-shaped inside wall of the channel-shaped recess (10) facing the container volume (5) and/or an essentially arc-shaped outside wall of the channel-shaped recess (10).

11. The container (1A, 1 B) for a cleaning or care medium according to any one of the preceding claims, **characterized by**
a top surface (11) which is connected to the at least one side wall (4A, 4B, 4C), wherein at least one section (11A) of the top surface (11) is arranged at an inclination to the bottom surface (2) and/or to the plane (3) along which the bottom surface (2) extends.

12. A cleaning or care device (12) for cleaning or care of an, in particular medical, dental or surgical instrument, having a container (1A, 1B) for a cleaning or care medium according to any one of the preceding claims and a conveyer device (13) for conveying the cleaning or care medium out of the container (1A, 1 B) and toward the instrument.

13. The cleaning or care device (12) for cleaning or care of an, in particular medical, dental or surgical instrument according to claim 12, **characterized by** an outside housing (14) having a base surface (15) and at least one side wall (16) connected thereto, a dispensing device (17) for dispensing a cleaning or care medium onto and/or into the instrument and at least one receptacle (18, 19) for the container (1A, 1 B) for a cleaning or care medium, wherein the at least one receptacle (18, 19) comprises a base surface (20A, 20B) for supporting the bottom surface (2) of the container (1A, 1B) and wherein the base surface (20A, 20B) extends along a plane (21), wherein the conveyor device (13) comprises a line section (13A-13C) which can be inserted into the outlet connector (6) of the container (1A, 1B), wherein the line section (13A-13C) which can be inserted into the outlet connector (6) of the container (1A, 1B) is arranged in the plane (21) along which the base surface (20A, 20B) extends or is arranged between the plane (21) along with the base surface (20A, 20B) extends and the base surface (15) of the outside housing (14).

14. The cleaning or care device (12) according to claim 13, **characterized in that** a setback (22), in particular in the form of a channel is provided in the base surface (20A, 20B) of the receptacle (18, 19) to receive the channel-shaped recess (10) of the container (1A, 1B), in particular to receive the protrusion or bulge (10A) of the container (1A, 1B) formed by the channel-shaped recess (10) on the outer surface (2B) of the bottom surface (2).

15. The cleaning or care device (12) according to claim 14, **characterized by** a depth (T2) of the setback (22) increasing in the direction of the line section (13A-13C) that can be inserted into the outlet connector (6) of the container (1A, 1 B).

## Revendications

1. Récipient (1A, 1B) pour un milieu de nettoyage ou d'entretien, en particulier pour le nettoyage ou l'entretien d'un instrument médical, dentaire, ou chirurgical, dans lequel le récipient (1A, 1 B) peut être introduit dans le logement d'un dispositif de nettoyage ou d'entretien et dans lequel le récipient (1A, 1 B) comprend: une surface de fond (2), dans lequel la surface de fond (2) s'étend le long d'un plan (3), au moins une paroi latérale (4A, 4B, 4C) reliée à la surface de fond (2), laquelle forme avec la surface de fond (2) un volume de récipient (5) pour le milieu de nettoyage ou d'entretien, et une tubulure de sortie (6) à travers laquelle il est possible de faire sortir le milieu de nettoyage ou d'entretien du récipient (1A, 1B), dans lequel la tubulure de sortie (6) est prévue sur cette au moins une paroi latérale (4A, 4B, 4C) et dans lequel le plan (3) le long duquel s'étend la surface de fond (2) traverse la tubulure de sortie (6) **caractérisé en ce que**
la tubulure de sortie (6) présente une ouverture de sortie (8) pour le milieu de nettoyage ou d'entretien espacée par rapport à cette au moins une paroi latérale (4A, 4B, 4C), dans lequel le plan (3) le long duquel s'étend la surface de fond (2) traverse l'ouverture de sortie (8).

2. Récipient (1A, 1B) pour un milieu de nettoyage ou d'entretien selon la revendication 1, **caractérisé en ce que**
la tubulure de sortie (6) comprend un axe central (7), l'axe central (7) et le plan (3) le long duquel s'étend la surface de fond (2) étant disposés sensiblement parallèlement entre eux.

3. Récipient (1A, 1 B) pour un milieu de nettoyage ou d'entretien selon la revendication 1 ou 2, **caractérisé en ce que**
la tubulure de sortie (6) comprend un axe central (7), dans lequel l'axe central (7) et le plan (3) le long duquel s'étend la surface de fond (2) sont disposés de manière décalée l'un par rapport à l'autre.

4. Récipient (1A, 1B) pour un milieu de nettoyage ou d'entretien selon l'une des revendications précédentes, **caractérisé en ce que**
l'axe central (7) de la tubulure de sortie (6) est disposé à peu près dans le plan (3), ou **en ce que** le plan (3) est disposé entre le volume de récipient (5) et l'axe central (7) de telle sorte que l'axe central (7) se situe en-dessous du plan (3) compte tenu du volume de récipient (5).

5. Récipient (1A, 1B) pour un milieu de nettoyage ou d'entretien selon l'une des revendications précédentes, **caractérisé en ce que**
l'on prévoit, sur la tubulure de sortie (6), en particulier sur l'ouverture de sortie (8) espacée par rapport à l'au moins une paroi latérale (4A, 4B, 4C), une fermeture (9), de préférence une membrane, pour la fermeture du récipient (1A, 1 B).

6. Récipient (1A, 1B) pour un milieu de nettoyage ou d'entretien selon l'une des revendications précédentes, **caractérisé en ce que**
l'on prévoit, sur la surface de fond (2), un creux en forme de goulotte (10) qui est relié à la tubulure de sortie (6).

7. Récipient (1A, 1B) pour un milieu de nettoyage ou d'entretien selon la revendication 6, **caractérisé en ce que**
la surface de fond (2) présente un côté intérieur (2A) tourné vers le volume de récipient (5) et un côté extérieur (2B), dans lequel le creux en forme de goulotte (10) forme, au niveau du côté extérieur (2B) de la surface de fond (2), une saillie (10A), en particulier un bombement.

8. Récipient (1A, 1 B) pour un milieu de nettoyage ou d'entretien selon la revendication 6 ou 7, **caractérisé par**
une profondeur (T1) du creux en forme de goulotte (10) qui augmente en direction de la tubulure de sortie (6).

9. Récipient (1A, 1B) pour un milieu de nettoyage ou d'entretien selon l'une des revendications 6 - 8, **caractérisé par**
une transition continue ou régulière du creux en forme de goulotte (10) dans la tubulure de sortie (6), en particulier par une transition continue ou régulière du paroi extérieure du creux en forme de goulotte (10) dans la paroi extérieure de la tubulure de sortie (6).

10. Récipient (1A, 1B) pour un milieu de nettoyage ou d'entretien selon l'une des revendications 6 - 8, **caractérisé par**
une paroi intérieure sensiblement en forme d'arc de cercle, tournée vers le volume de récipient (5), du creux en forme de goulotte (10) et/ou une paroi extérieure sensiblement en forme d'arc de cercle du creux en forme de goulotte (10).

11. Récipient (1A, 1B) pour un milieu de nettoyage ou d'entretien selon l'une des revendications précédentes, **caractérisé par**
une surface de recouvrement (11) reliée à l'au moins une paroi latérale (4A, 4B, 4C), dans lequel au moins une partie (11A) de la surface de recouvrement (11) est disposée de manière inclinée vers la surface de fond (2) et/ou le plan (3) le long duquel s'étend la surface de fond (2).

12. Dispositif de nettoyage ou d'entretien (12) pour le nettoyage ou l'entretien d'un instrument en particulier médical, dentaire, ou chirurgical, avec un récipient (1A, 1 B) pour un milieu de nettoyage ou d'entretien selon l'une des revendications précédentes et un dispositif d'acheminement (13) pour l'acheminement du milieu de nettoyage ou d'entretien hors du récipient (1A, 1 B) jusque vers l'instrument.

13. Dispositif de nettoyage ou d'entretien (12) pour le nettoyage ou l'entretien d'un instrument en particulier médical, dentaire, ou chirurgical selon la revendication 12, **caractérisé par**
un boîtier extérieur (14) avec une surface de base (15) et au moins une paroi latérale (16) reliée à celle-ci, un dispositif de délivrance (17) pour la délivrance d'un milieu de nettoyage ou d'entretien sur l'instrument et/ou dans l'instrument, et par au moins un logement (18, 19) pour le récipient (1A, 1B) pour un milieu de nettoyage ou d'entretien, dans lequel l'au moins un logement (18, 19) présente une surface de fond (20A, 20B) pour la mise en place de la surface de fond (2) du récipient (1A, 1B), et dans lequel la surface de fond (20A, 20B) s'étend le long d'un plan (21), dans lequel le dispositif d'acheminement (13) présente une partie de conduction (13A - 13C) pouvant être introduite dans la tubulure de sortie (6) du récipient (1A, 1B), dans lequel la partie de conduction (13A - 13C) pouvant être introduite dans la tubulure de sortie (6) du récipient (1A, 1 B) est disposée dans le plan (21) le long duquel s'étend la surface de fond (20A, 20B), ou entre le plan (21) le long duquel s'étend la surface de fond (2) et la surface de base (15) du boîtier extérieur (14).

14. Dispositif de nettoyage ou d'entretien (12) selon la revendication 13, **caractérisé en ce que**
l'on prévoit, dans la surface de fond (20A, 20B) du logement (18, 19), un retrait (22) en particulier en forme de goulotte pour la réception du creux en forme de goulotte (10) du récipient (1A, 1B), en particulier pour la réception de la saillie ou du bombement (10A) formé(e) par le creux en forme de goulotte (10) sur le côté extérieur (2B) de la surface de fond (2) du récipient (1A, 1 B).

15. Dispositif de nettoyage ou d'entretien (12) selon la revendication 14, **caractérisé par** une profondeur (T2) du retrait (22) augmentant en direction de la partie de conduction (13A - 13C) pouvant être introduite dans la tubulure de sortie (6) du récipient (1A, 1 B).
